(19) Europäisches Patentamt
European Patent Office
Office européen des brevets

(11) Publication number: **0 164 920 B1**

(12) **EUROPEAN PATENT SPECIFICATION**

(45) Date of publication of patent specification: **28.11.90**

(51) Int. Cl.$^5$: **C 08 G 77/54,** H 01 L 21/56, H 01 L 23/29

(21) Application number: **85303431.2**

(22) Date of filing: **15.05.85**

(54) **A soluble imide oligomer and a method for producing the same.**

(30) Priority: **15.05.84 JP 97187/84**

(43) Date of publication of application:
**18.12.85 Bulletin 85/51**

(45) Publication of the grant of the patent:
**28.11.90 Bulletin 90/48**

(84) Designated Contracting States:
**CH DE FR GB LI**

(56) References cited:
**GB-A-2 101 148**
**GB-A-2 123 017**

**PATENT ABSTRACTS OF JAPAN, vol. 7, no. 273 (C-198)1418r, 6th December 1983; & JP - A - 58 152 018 (HITACHI KASEI KOGYO K.K.) 09-09-1983**

**PATENT ABSTRACTS OF JAPAN, vol. 7, no. 77 (C-159)1222r, 30th March 1983; & JP - A - 58 7473 (HITACHI KASEI KOGYO K.K.) 17-01-1983 (Cat. D,A)**

(73) Proprietor: **Chisso Corporation**
**6-32, Nakanoshima 3-chome Kita-ku**
**Osaka-shi Osaka-fu (JP)**

(72) Inventor: **Kunimune, Kouichi**
**No. 2 of 12, Ichihara**
**Ichiharashi Chibaken (JP)**
Inventor: **Konotsune, Shiro**
**No. 6 of 12, Highland 5-chome**
**Yokosukashi Kanagawaken (JP)**

(74) Representative: **Ruffles, Graham Keith et al**
**MARKS & CLERK 57-60 Lincoln's Inn Fields**
**London WC2A 3LS (GB)**

**EP 0 164 920 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

PATENT ABSTRACTS OF JAPAN, vol. 7, no. 289 (C-202)1434r, 23rd December 1983; & JP - A - 58 168 624 (NITTO DENKI KOGYO K.K.) 05-10-1983

PATENT ABSTRACTS OF JAPAN, vol. 6, no. 244 (C-138)1122r, 2nd December 1982; & JP - A - 57 143 328 (NITTO DENKI KOGYO K.K.) 04-09-1982 (Cat. D,A)

PATENT ABSTRACTS OF JAPAN, vol. 7, no. 83 (C-160)1228r, 7th April 1983; & JP - A - 58 13631 (HITACHI KASEI KOGYO K.K.) 26-01-1983 (Cat. D,A)

PATENT ABSTRACTS OF JAPAN, vol. 6, no. 40 (C-94)918r, 12th March 1982; & JP - A - 56 157 428 (NITTODENKI KOGYO K.K.) 04-12-1981

**Description**

Background of the Invention

Field of Art

This invention relates to a novel precursor of a silicon-containing polyimide polymer, a method for preparing the same, and a use of the same.

As protective and insulating materials in the field of electronics, polyimide resins have been widely used mainly from the viewpoint of their heat-resisting properties. From the overall point of view, however, they are not always satisfactory in this field with not a few points to be improved. In general, a precursor of polyimide for electronic materials is coated, in the form of a polyamic acid solution, on a base material, and baked to form a cured imide product. Such a coating solution has extremely high viscosity, leading to a drawback of poor workability. Furthermore, such a precursor has other drawbacks that baking thereof requires so high temperatures, e.g., 300 to 400°C, as to exceed the temperature of heat resistance of the base material, and that adhesion thereof to an object to be coated therewith, such as a silicon wafer or glass is insufficient.

Prior Arts

A number of copolymers of polyimide precursors with silicon compounds have been proposed for the improvement of adhesion. For example, Japanese Patent Application Laid-Open Specifications No. 143,328 (1982), No. 7,473 (1983) and No. 13,631 (1983) have proposed polyimide-siloxane copolymers which are obtained by replacing a part of a diamine as a raw material with a polysiloxane terminated with diamine molecules at both the ends thereof. However, this case involves drawbacks that the heat-resisting properties of cured products formed from these copolymers are reduced with increasing siloxane contents in the copolymers, and that copolymerization of the diamine with the polysiloxane entails lowering of the polymerization degree of the resulting polyamic acid, leading to poor film forming properties.

Japanese Patent Publications No. 32,162 (1983), and No. 32,163 (1983) disclose polyamide carboxylic acids and the like including amic acids usable in the present invention. However, these polyamide carboxylic acids and the like have a drawback that baking thereof into imides requires high temperature of at least 300°C as in the case of conventional polyamide carboxylic acids. These pieces of patent literature further disclose cyclization (imide formation) carried out chemically by a treatment with a dehydrating agent under mild conditions. However, the cyclization according to this treatment requires a long time, and the resultant cyclized products have a drawback that those having high Si contents are inferior in film forming properties due to low viscosities in the form of varnishes thereof attributed to low molecular weights thereof, while those having low Si contents are inferior in adhesion of cured products therefrom to a silicon wafer or glass.

It is, therefore, an object of the present invention to provide a polyimide-siloxane prepolymer which are suitable for use in surface protecting films for semi-conductors and in insulating films between multilayer wiring layers, soluble in an adequate solvent, capable of being cured by baking a relatively low temperature, which provides good workability when made into a solution and good film-forming properties, and which provides good adhesion of a cured product therefrom to a silicon wafer, glass, and the like.

Summary of the Invention

In accordance with one aspect of the present invention, there is provided a soluble imide oligomer having an inherent viscosity of 0.05 to 0.5 dl/g, and represented by a general formula (I) mentioned below.

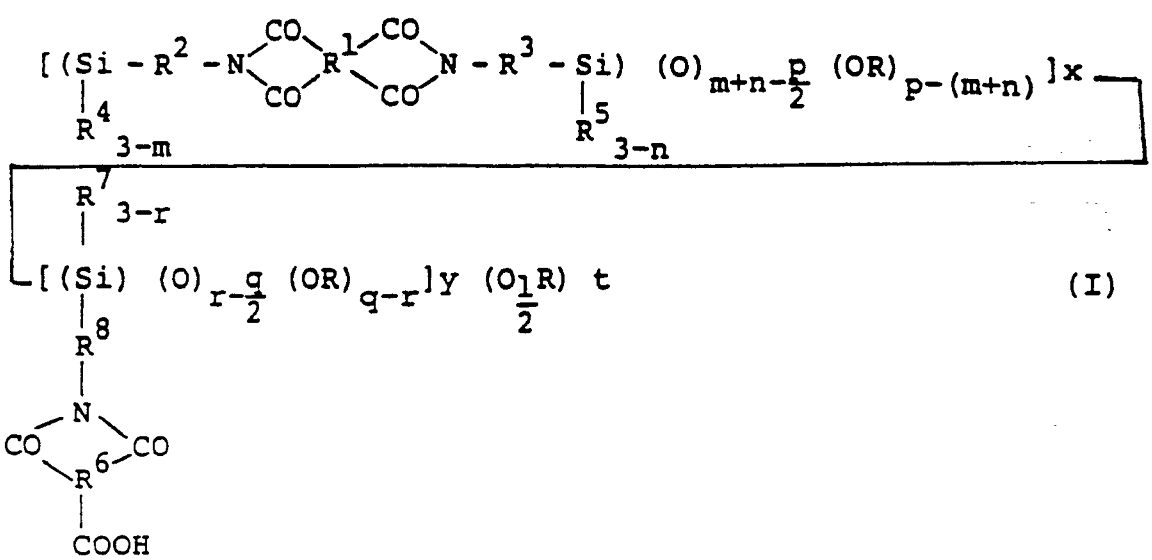

(I)

In accordance with another aspect of the present invention, there is provided a process for preparing a soluble imide oligomer of the kind as defined above, which comprises reacting an amic acid compound represented by a general formula (II) mentioned below, or a mixture thereof with 10 mole% or less of an amic acid compound represented by a general formula (III) mentioned below, based on the total amount of the compounds (II) and (III), in the presence of 70% by weiight or more, based on a solution, of a solvent, at a temperature of 60 ~ 200°C for 0.2 ~ 6 hours, and, if necessary, in the additional presence of {(m + n − 2)x + (r − 1)y} mole or less, per mole of the above-mentioned amic acid compound or mixture, of water at a temperature of 60 to 200°C for 0.2 to 6 hours.

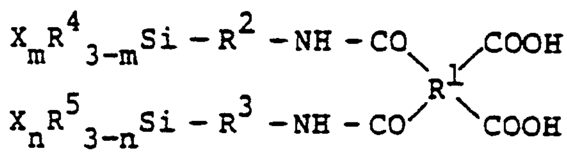 (II)

$$X_rR^7{}_{3-r}Si-R^8-NH-CO-R^6-(COOH)_2 \qquad \text{(III)}$$

In accordance with still another aspect of the present invention, there is provided a coating solution for electronic materials comprising a solvent and 30% by weight or less, based on the total of said solvent and the following oligomer, of a soluble imide oligomer of the kind as defined above.

In the above-mentioned formulae (I), (II) and (III), $R^1$ and $R^6$ are tetravalent and trivalent carbocyclic aromatic groups, respectively, carbonyl groups in carboxyl, amide and imide groups being bonded to the carbocyclic aromatic groups at the mutually different ring-forming carbon atoms therof, every pair of imide ring-forming carbonyl groups being bonded to a carbocyclic ring of the carbocyclic aromatic groups in the mutual ortho positions thereof; $R^2$, $R^3$ and $R^8$, which are the same or different groups, are

$-(CH_2)_s-$, 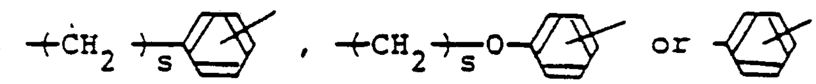

(wherein s is an integer of 1 to 4); $R^4$, $R^5$ and $R^7$, which are the same or different groups, are an alkyl group having 1 to 6 carbon atoms, a phenyl group, or an alkyl-substituted phenyl group having 7 to 12 carbon atoms; X is an alkoxyl group, an acetoxyl group, a halogen atom, or a hydroxyl group; R is hydrogen or alkyl or acetyl group derived from X; m and n each are an integer of 1, 2 or 3, and satisfy an equation $m + n \geqq 3$; r is 2 or 3; p and q are integers and satisfy the relation of $m + n \leqq p < 2(m + n) - 1$ and $r \leqq q < 2r - 1$; t is 0 or 2; and x and y correspond to relative amounts (by mole) of the amic acid compounds reacted, and satisfy an equation;

$$0 \leqq \frac{100\,y}{x + y} \leqq 10 \text{ preferably } 0.1 \leqq \frac{100\,y}{x + y} \leqq 10$$

The above-mentioned inherent viscosity is "η inh" represented by the following equation:

$$\eta\ inh = \frac{\ln \eta/\eta_0}{C}$$

wherein η is a value as measured using an Ubbellohde viscometer at a sample concentration of 0.5 g/dl in a solvent, N-methyl-2-pyrrolidone, at a temperature of 30 ± 0.01°C, $\eta_0$ is a value for the solvent, N-methyl-2-pyrrolidone, as measured using the same viscometer at the same temperature, and C represents the sample concentration of 0.5 g/dl.

As examples of the above-mentioned

$R^1$, 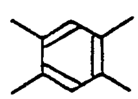 , 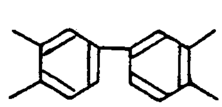 and 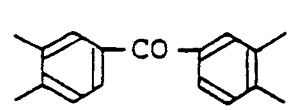

can be mentioned and as examples of the above-mentioned $R^4$, $R^5$ and $R^7$, $-CH_3$, $-C_2H_5$ and

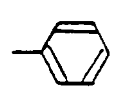

can be mentioned.

Examples of the compound represented by the general formula (II) and to be used in the process of the present invention include the following compounds.

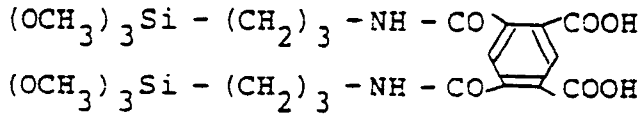

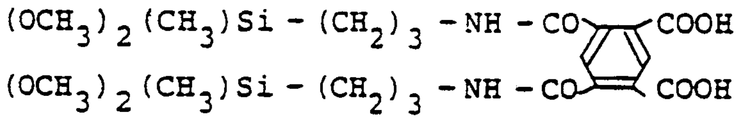

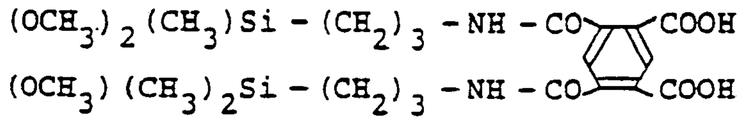

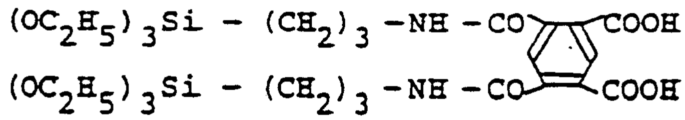

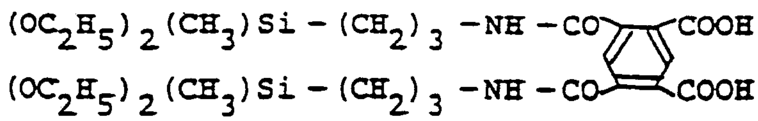

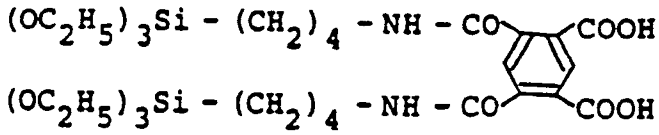

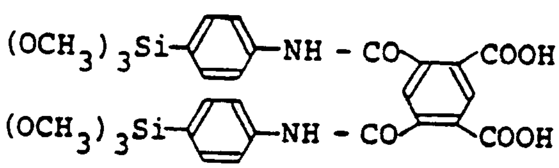

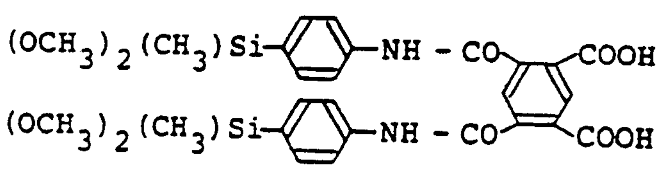

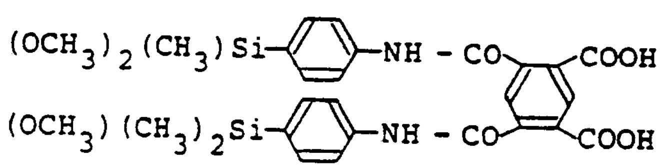

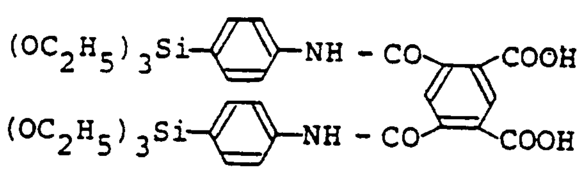

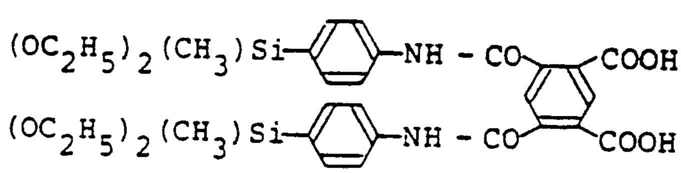

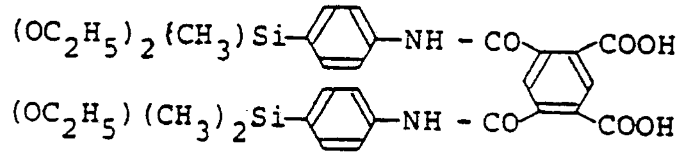

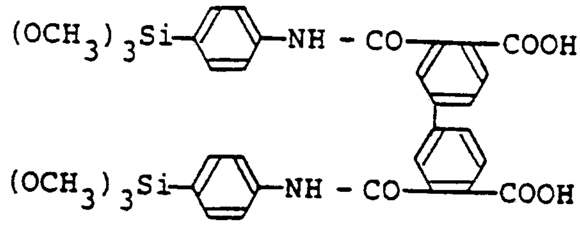

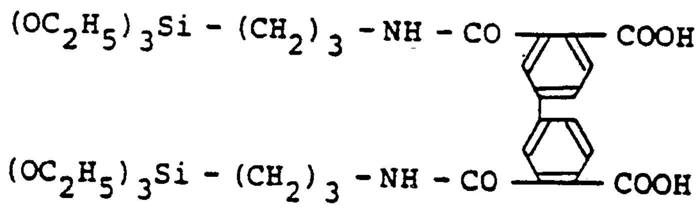

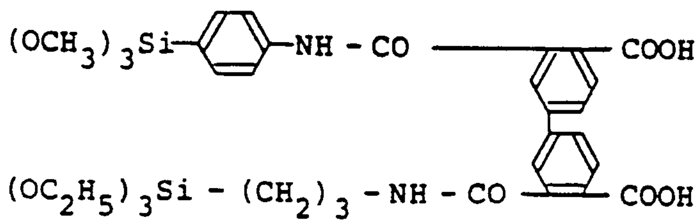

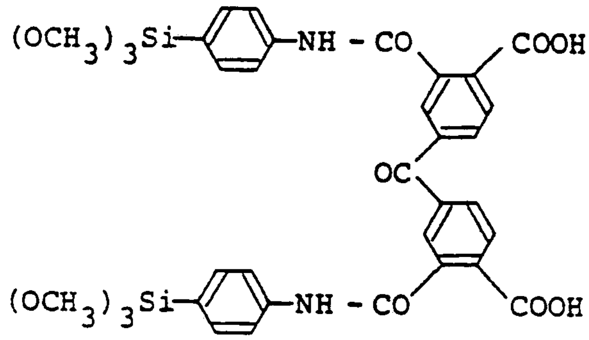

These amic acid compounds can be prepared, for example, by reacting a tetracarboxylic dianhydride with amino silicon compounds, respectively represented by general formulae (IV) and (V) mentioned below:

$$NH_2—R^2—SiR^4_{3-m}X_m \qquad (IV)$$

$$NH_2—R^3—SiR^5_{3-n}X_n \qquad (V)$$

wherein $R^2$, $R^3$, $R^4$, $R^5$, X, m and n have the same meanings as defined before. In the reaction, the amino silicon compounds of the formulae (IV) and (V) may be either the same or different.

As an example of the above-mentioned $R^6$,

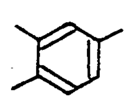

Examples of the compound represented by the general formula (III) and to be used in the process of the present invention include the following compounds.

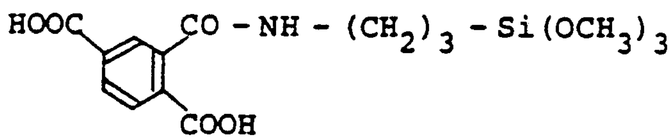

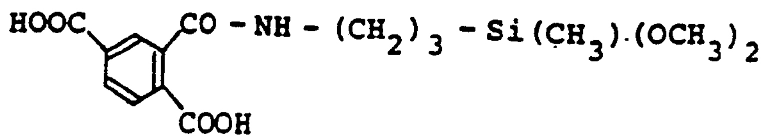

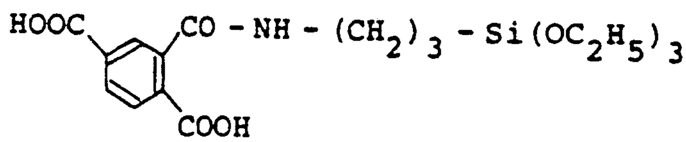

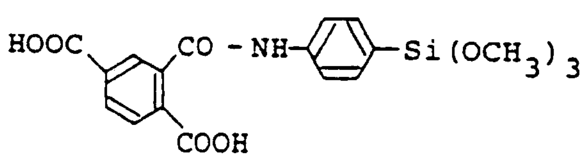

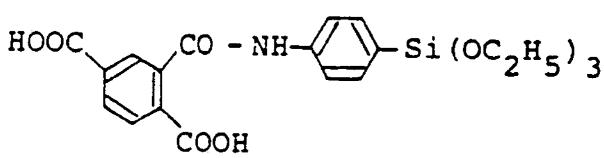

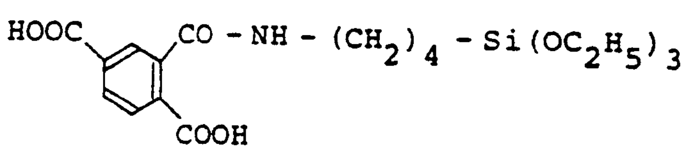

These amic acid compounds can be prepared, for example, by reacting a tricarboxylic acid anhydride with an amino silicon compound represented by a general formula (VI) mentioned below.

$$NH_2—R^8—SiR^7_{3-r}X_r \qquad (VI)$$

wherein $R^7$, $R^8$, X and r have the same meanings as defined before.

The imide oligomer compound of the present invention may be synthesized by reacting the aforementioned amic acid compound (II), or compounds (II) and (III) in the presence of a solvent as mentioned later under the specifically controlled conditions.

If the aforementioned amic acid compound (III) is involved in the synthesis, the imide oligomer according to the present invention provides improved adhesion to a metal when it is coated on the metal. The compound (III) content of the reaction system is 10% by mole or less based on the total amount of the compounds (II) and (III). Too high a compound (III) content may disadvantageously result in poor film forming properties of an imide oligomer to be obtained. When the compound (III) content of the reaction system is about 0.1% by mole or more based on the total amount of the compounds (II) and (III), there appears a marked effect on improvement in adhesion of the imide oligomer according to the present invention to a metal. However, any content of the compound (III) lower than the above-mentioned is permissible for the purpose of the present invention.

Specific examples of the reaction solvent include N-methyl-2-pyrrolidone, dimethylacetamide, dimethylformamide, dimethyl sulfoxide, tetramethylurea, pyridine, dimethylsulfone, hexamethylphosphoric amide, methylformamide, N-acetyl-2-pyrrolidone, toluene, xylene,

methoxyethanol, and 2-ethoxy-ethanol. They may be used either alone or in combination. A mixed solvent of 30 to less than 100% by weight of at least one of these solvent with at least one different solvent may also be used. The specifically controlled reaction conditions mentioned before will be explained as follows. An amic acid compound of the general formula (II), or a combination of amic acid compounds of the rerspective general formulae (II) and (III) is dissolved in the above-mentioned solvent. The amic acid concentration is preferably 30% by weight or less. When the concentration exceeds 30% by weight, there is a fear of formation of some non-dissolved imide compound though it depends on the conditions. The lower limit of the amic acid compound concentration should be determined with the procedure of using the resultant product and the economical viewpoint being taken into consideration. The lower limit is preferably about 2% by weight.

The reaction temperature is preferably 60 to 200°C. Temperatures of less than 60°C may allow the reaction to proceed, but sometimes leads to uneconomical result. A temperature exceeding 200°C is unnecessary. A reaction time of 0.2 to 6 hours is satisfactory.

When the reaction is carried out under the conditions as described above, the amic acid compound of the general formula (II), or the amic acid compounds of the general formulae (II) and (III) are subjected to dehydration to form an imide oligomer compound.

If X in the foregoing formulae is a hydrolizable group such as an alkoxyl group, hydrolysis, occurs with water formed by the imide formation reaction, followed by condensation to form siloxane linkages allowing polymerization to proceed. Too much increased a molecular weight of the product entails insolubilization thereof in the solvent. On the contrary, when hydrolysis and condensation reactions are not allowed to proceed, an imide oligomer compound having no or poor film forming properties is obtained. More specifically, a soluble imide oligomer having good film forming properties can be obtained only under the controlled reaction conditions in accordance with the process of the present invention. An imide formation reaction promoting agennt such as a tertiary amine may be added to the reaction system, but is not always required. This is because, in the case of X being a hydrolizable group, water formed by imide formation reaction is immediately consumed for hydrolysis of X under the reaction conditions adopted in the present invention to enable the imide formation reaction to proceed mildly but rapidly as compared with the usual imide formation reactions. This is one feature of the process of the present invention.

The hydrolysis reaction can be promoted with the aid of an acid catalyst, which may, however, give a bad influence when remained after the reaction. In view of this, if the hydrolysis reaction insufficiently proceeds, the reaction is preferably allowed to proceed with addition of $\{(m + n - 2)x + (r - 1)y\}$ mole or less, per mole of the amic acid compound(s), of water, without addition of any aciid catalyst subsequently to the reaction (having been carried out under the condition without addition of water) at the same temperature and for the same time. Thus the desired imide oligomer can be obtained.

The imide compound of the present invention thus synthesized is an oligomer having an inherent viscosity of 0.05 to 0.5 dl/g as measured under the following conditions.

$$\text{Inherent Viscosity } \eta \text{ inh} = \frac{\ln \eta/\eta_0}{C}$$

The measurement is carried out using an Ubbellohde viscometer at a sample concentration of 0.5 g/dl in a solvent of N-methyl-2-pyrrolidone, at a temperature of 30 ± 0.01°C. Herein, $\eta$ and $\eta_0$ are viscosities of a sample solution of 0.5 g/dl in concentration and the solvent, respectively, and C represents the sample concentration of 0.5 g/dl.

In the case of an imide compound of less than 0.05 dl/g in inherent viscosity, a coating solution thereof is not satisfactory in film forming properties. On the other hand, such an imide oligomer compound as has an inherent viscosity exceeding 0.5 dl/g cannot be measured with respect to accurate inherent viscosity because of poor solvent solubility thereof.

If F is substituted for $R^2$

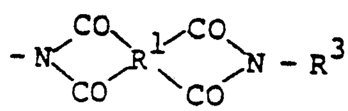

and G is substituted for

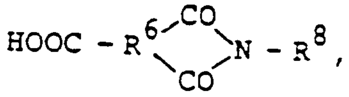

one part of the oligomer of the present invention can be expressed for example as follows:

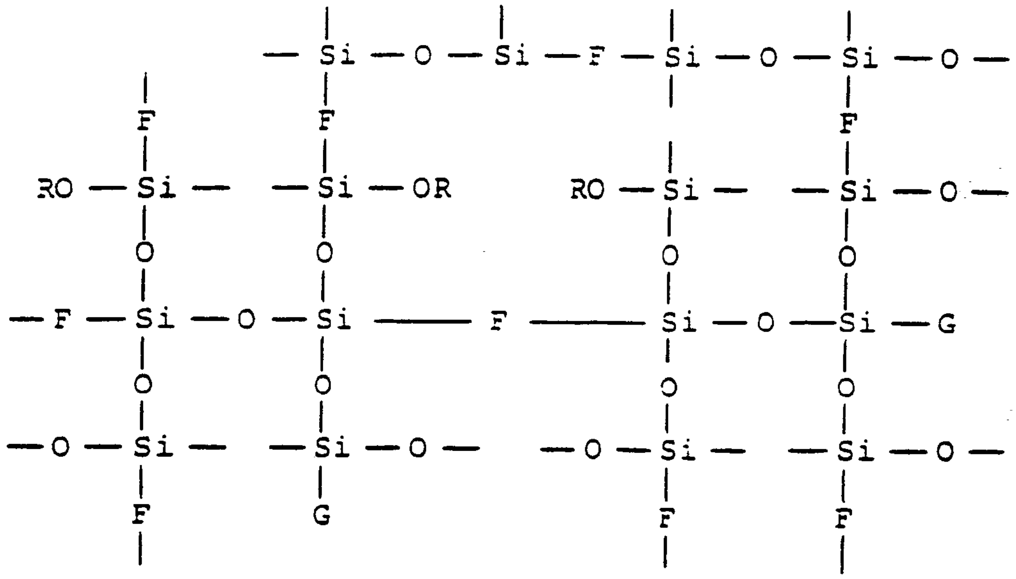

In case the imide oligomer compound of the present invention is used in the form of a coating solution for electronic materials, it may, according to need, be rid of any ionic compounds by adsorption with a solid adsorbent, or other procedure, and/or of minute solid impurities with filtration with a filter of 0.5 μm or less in mesh size. The aforementioned reaction solvent may be used as the solvent of the coating solution, too. The imide oligomer concentration of the coating solution may be determined depending on the desired thickness of a coated film, but is preferably 30% by weight or less, more preferably 0.3 to 25% by weight, based on the total of the solvent and the oligomer, form the practical point of view. Such an imide oligomer concentration as exceeds 30% by weight based on the total of the solvent and the oligomer disadvantageously results in poor flowability. Besides the compounds of the general formula (I), the coating solution may additionally comprise an oligomer apparently represented by the same formula as the general formula (I) but defined with such different meanings of x and y in the formula as satisfies the equation: $100\ y/(x + y) > 10$ (including a case of $x = 0$). Also in this case, the amount of the compound of the formula (III) is preferably 10% by mole or less based on the total amount of the compounds of the formulae (II) and (III) in order not to give harmful effect upon insulating property. The coating solution may be uniformly coated on a silicon wafer, a glass plate or other material according to any customary procedure, for example, using a spinner, followed by baking to form a hard film. The baking conditions may be more or less different depending on the solvent used, the thickness of a film formed and the like. A baking temperature of 100 to 300°C, and a baking time of 0.5 to 1.5 hours are satisfactory.

The merits of the present invention will be specifically described. A coating solution for electronic materials which contains the imide oligomer compound according to the present invention is compared with a conventional coating solution of a precursor of polyimide. Since it contains a polymer of an amic acid, the latter solution requires a high baking temperature, is subject to a large change with time, and presents a difficulty in handling due to its high viscosity. On the other hand, since the coating solution according to the present invention contains a compound already subjected to an imide formation reaction, the baking temperature thereof may be low as compared with that of the conventional coating solution, and the handling thereof is easy due to low viscosity of the solution, which can be attributed to a low molecular weight of the oligomer compound. Furthermore, since the oligomer compound contains many siloxane linkages, a coated film formed from the coating solution according to the present invention has good adhesion to a base material such as a silicon wafer or glass.

An imide oligomer according to the present invention synthesized using an amic acid compound of the general formula (III) as a part of the raw materials has improved adhesion to metals such as copper, and aluminum, and, hence, a highly practical merit.

With respect to heat resisting properties, a coated film formed from a coating solution containing the reacton product of only aromatic amic acid compound(s) according to the present invention is never inferior to a coated film formed from the conventional coating solution comprising only other aromatic polyamic acid(s). Furthermore, the coated film formed from the coating solution according to the present invention turned out to be capable of being widely changed in surface hardness depending on the kind of amic acid compound(s) chosen as the raw material(s).

As described above, great industrial merits can be gained by providing the compound of the present

invention having a variety of excellent properties and the process of the present invention for preparing the same easily and efficiently.

Preferred Embodiments of the Invention

The following Examples illustrate the present invention in connection with the merits thereof in more detail, but should not be construed as limiting the scope thereof.

Reference Example 1

A 1 l flask equipped with an agitator, a dropping funnel, a thermometer and a nitrogen replacement means was set in cool water. After the atmosphere in the flask was replaced with nitrogen, 500 ml of N-methyl-2-porrolidone as dehydrated and purified and 185.58 g (0.838 mole) of 3-aminopropyltriethoxysilane were put into the flask, followed by agitation. 91.42 g (0.419 mole) of pyromellitic dianhydride in the form of a powder was put into the dropping funnel, from which the powder was fallen inside the flask little by little over 30 minutes to continue a reaction. During the 30 minutes' period, the reaction temperature was maintained in the range of 0 to 5°C. After that, the temperature was raised to 20 to 25°C, in which range the reaction was continued for one hour. A nearly colorless and transparent liquid was obtained.

A part of the reaction mixture solution was sampled and dried under room temperatures and reduced pressures. The light-brown substance thus obtained was subjected to infrared absorption spectroscopic analysis. Absorption peaks assigned to amide appeared at 3,280 $cm^{-1}$ and 1,550 $cm^{-1}$, while an absorption peaks at 1,490 $cm^{-1}$ assigned to amine in a raw material compound disappeared. An absorption peak at 1,780 $cm^{-1}$ assigned to carbonyl in pyromellitic dianhydride as another raw material compound shifted to 1,720 $cm^{-1}$. These clearly indicate formation of an amic acid compound.

Reference Example 2

Using the same equipment and substantially the same procedure as in Reference Example 1, 85.19 g (0.399 mole) of p-aminophenylmethoxysilane and 43.56 g (0.20 mole) of pyromellitic dianhydride were reacted in 500 ml of n-methyl-2-pyrrolidone as the solvent at 5 to 15°C for one hour and at 20 to 25°C for further one hour.

As a result of infrared absorption spectroscopic analysis of the reaction product, formation of an amic acid compound was confirmed in the same manner as in Reference Example 1.

Reference Example 3

Using the same equipment and substantially the same procedure as in Reference Example 1, 82.02 g (0.429 mole) of 3-aminopropylmethyldiethoxysilane and 46.73 g (0.214 mole) of pyromellitic dianhydride were reacted in 500 ml of N-methyl-2-pyrrolidone as the solvent at 0 to 10°C for one hour and at 20 to 25°C for further two hours.

As a result of infrared absorption spectroscopic analysis of the reaction product, formation of an amic acid compound was confirmed in the same manner as in Reference Example 1.

Reference Example 4

Using the same equipment and substantially the same procedure as in Reference Example 1, 82.50 g (0.460 mole) of 3-aminopropyltrimethoxysilane and 74.17 g (0.0230 mole) of 3,3',4,4'-benzophenonetetracarboxylic dianhydride were reacted in 500 ml of N,N-dimethylacetamide at 5 to 10°C for one hour and at 25 to 30°C for further two hours.

As a result of infrared absorption spectroscopic analysis of the reaction product, formation of an amic acid compound was confirmed in the same manner as in Reference Example 1.

Reference Example 5

Using the same equipment and substantially the same procedure as in Reference Example 1, 79.3 g (0.360 mole) of 3-aminopropyltrimethoxysilane and 39.27 g (0.180 mole) of pyromellitic dianhydride were reacted in 500 ml of N,N-dimethylformamide at 0 to 5°C for one hour and at 20 to 25°C for further one hour.

As a result of infrared absorption spectroscopic analysis of the reaction product, formation of an amic acid compound was confirmed in the same manner as in Reference Example 1.

Reference Example 6

Using the same equipment and substantially the same procedure as in Reference Example 1, 68.93 g (0.311 mole) of 3-aminopropyltriethoxysilane and 59.82 g (0.311 mole) of trimellitic dianhydride were reacted in 500 ml of N-methyl-2-pyrrolidone at 5 to 10°C for one hour and at 25 to 30°C for further three hours.

As a result of infrared absorption spectroscopic analysis of the reaction product, formation of an amic acid compound was confirmed in the same manner as in Reference Example 1.

Subsequently, imide oligomers according to the present invention were synthesized using the amic acid compounds prepared in Reference Examples, in some of the following Examples, which will give better understanding of the invention in conjunction with the accompanying drawing in which:

Fig. 1 is an infrared absorption spectra chart for an imide oligomer according to the present invention, which was prepared in Example 1.

Example 1

A 100 ml flask equipped with an agitator, a thermometer and a nitrogen replacement means was set in a constant temperature bath. After the atmosphere in the flask was replaced with nitrogen, 14.29 g of the 35% by weight solution of the amic acid compound synthesized in Reference Example 1 and 35.71 ag of N-methyl-2-pyrrolidone were put into the flask to prepare 50 g of a 10% by weight solution. The solution thus prepared was subjected, with agitation, to a reaction at 150°C for 3 hours. A light-yellow and transparent liquid was obtained.

A part of the reaction mixture solution was sampled and dried under room temperatures and reduced pressures. A light-brown solid thus obtained was subjected to infrared absorption spectroscopic analysis. The chart obtained is shown in Fig. 1, from which it is apparent that absorption peaks at 3,280 $cm^{-1}$ and 1,550 $cm^{-1}$ asigned to amide disappeared while new peaks at 1,780 $cm^{-1}$ and 720 $cm^{-1}$ assigned to imide ring appeared. This clearly indicates that the amic acid compound was dehydrated to form an imide compound. The inherent viscosity (η inh) of this compound was 0.21 dl/g.

Comparative Example 1

Using the same equipment and substantially the same procedure as in Example 1, 50 g of the 35% by weight solution of the amic acid compound synthesized in Reference Example 1 was subjected, without further dilution with any solvent, to a reaction carried out by gradually raising the temperature from room temperatures. 40 minutes after start of heating, when the temperature reached 140°C, the solution was gelled and lost its flowability.

Comparative Example 2

Using the same equipment and substantially the same procedure as in Example 1, 50 g of a 10% by weight solution was prepared from 14.29 g of the 35% by weight solution of the amic acid compound synthesized in Reference Example 1 and 35.71 g of N-methyl-2-pyrrolidone. The solution thus prepared was subjected, in the presence of 1.0 g of pyridine as a catalyst and 2.0 g of acetic anhydride as a dehydrating agent for promoting imide formation, to a reaction at 40°C for 10 hours.

As a result of infrared absorption spectroscopic analysis, the compound thus obtained was confirmed to be an imide compound. The inherent viscosity of this compound was 0.022 dl/g.

Example 2

Using the same equipment and substantially the same procedure as in Example 1, 50 g of the 20% by weight solution of the amic acid compound synthesized in Reference Example 2 was as such subjected to a reaction at 80°C for 3 hours. A light-brown and transparent liquid was obtained.

As a result of infrared absorption spectroscopic analysis of the reaction product, formation of an imide compound was confirmed. The inherent viscosity of this compound was 0.083 dl/g.

Example 3

Using the same equipment and substantially the same procedure as in Example 1, 50 g of a 5% by weight solution was prepared from 12.5 g of a 20% by weight solution of the amic acid compound synthesized in Reference Example 3 and 37.5 g of N-methyl-2-pyrrolidone. The solution thus prepared was subjected, with addition of 0.25 g of water for promoting hydrolysis, to a reaction at 180°C for 5 hours.

As a result of infrared absorption spectroscopic analysis, the compound thus obtained was confirmed to be an imide compound. The inherent viscosity of this compound was 0.39 dl/g.

Example 4

Using the same equipment and substantially the same procedure as in Example 1, 50 g of the 25% by weight solution of the amic acid compound synthesized in Reference Example 4 was as such subjected to a reaction at 60°C for 5 hours.

As a result of infrared absorption spectroscopic analysis, the compound thus obtained was confirmed to be an imide compound. The inherent viscosity of this compound was 0.22 dl/g.

Example 5

Using the same equipment and substantially the same procedure as in Example 1, 50 g of a 10% by weight solution was prepared from 25 g of the 20% by weight solution of the amic acid comound synthesized in Reference Example 5 and 25 g of methyl cellosolve. The solution thus prepared was subjected to a reaction at 100°C for 3 hours.

As a result of infrared absorption spectroscopic analysis, the compound thus obtained was confirmed to be an imide compound. The inherent viscosity of this compound was 0.13 dl/g.

Example 6

Using the same equipment as used in Example 1, substantially the same procedure as in Example 1

was repeated. More specifically, 13.90 g of the 35% by weight solution of the amic acid compound synthesized in Reference Example 1, 0.68 g of the 20% by weight solution of the amic acid compound synthesized in Reference Example 6, and 35.42 g of N-methyl-2-pyrrolidone were put into the flask to prepare 50 g of a solution having a total amic acid compounds concentration of 10% by weight and a proportion of the amic acid compound represented the general formula (III) prepared from trimellitic acid anhydride and 3-aminopropyltriethoxysilane, relative to the total amic acid compounds, of 5.0% by mole, which was then subjected, with agitation, to a reaction at 100°C for 5 hours. A light-yellow and transparent liquid was obtained.

As a result of infrared absorption spectroscopic analysis of the reaction product, formation of an imide compound was confirmed. The inherent viscosity of this compound was 0.078 dl/g.

Example 7

The following coating tests were conducted. The reaction mixture solutions prepared in Reference Examples, Examples and Comparative Examples were filtrated with a 0.2 μm filter, and coated on glass plates, followed by baking at 100 to 300°C for one hour. The states of coated films thus formed were observed.

The results are shown in Table 1.

Table 1

| Run No. | Coating Solution | Baking Temperature | | | State of Coated Film |
|---|---|---|---|---|---|
| | | 100°C | 200°C | 300°C | |
| 1 | Reference Example 1 | × | × | × | A film was not formed. |
| 2 | Reference Example 2 | Δ | Δ | × | A film was formed by baking at 100 to 200°C, but poor in strength. |
| 3 | Reference Example 3 | Δ | Δ | × | ditto |
| 4 | Reference Example 4 | × | × | × | A film was not formed. |
| 5 | Reference Example 5 | × | × | × | ditto |
| 6 | Reference Example 6 | × | × | × | ditto |
| 7 | Comparative Example 2 | × | × | × | ditto |
| 8 | Example 1 | o | o | o | A sufficiently strong film was formed by baking at 100 to 300°C. Its adhesion to a glass plate was good. |
| 9 | Example 2 | o | o | o | ditto |
| 10 | Example 3 | o | o | o | ditto |
| 11 | Example 4 | o | o | o | ditto |
| 12 | Example 5 | o | o | o | ditto |
| 13 | Example 6 | o | o | o | ditto |

× A film was not formed. Δ A film was formed, but poor in strength.

o A sufficiently strong film was formed.

Example 8

The following adhesion tests were conducted. Every coating solution as indicated in Table 2 was coated on the surfaces of a silicon dioxide substrate and a copper plate by means of a spinner, followed by baking at 200°C for one hour, to form, respective coated films of 1 to 2 μm in thickness. Each coated film was subjected to such cutting as to form cut lines dividing the film into a number of squares of 2 mm in length of a side thereof. A cellophane adhesive tape was pressed to adhere to the surfaces of the squares, followed by immediate strippng-off of the tape therefrom. The number of squares stripped off together with the tape from the substrate or plate was counted, and expresed in terms of the number thereof per 100 original squares. The results are shown in Table 2.

Table 2

| Run No. | Coating Solution | Kind of Substrate | |
|---|---|---|---|
| | | Silicon Dioxide | Copper |
| 1 | Example 1 | 0 | 87 |
| 2 | Example 6 | 0 | 0 |
| 3 | Comparative *1 Example 3 | 100 | 100 |

*1: A solution of a conventional precursor of polyimide (polyamic acid solution) prepared by reacting an equimolar mixture of pyromellitic dianhydride and diaminodiphenyl ether in N-methyl-2-pyrrolidone, Solids Content: 15% by weight, Inherent Viscosity: 0.82 dl/g as measured in the aforementioned manner.

**Claims**

1. A soluble imide oligomer having an inherent viscosity (η inh) of 0.05 to 0.5 dl/g as determined by the following equation:

$$\eta\ inh = \frac{\ln \eta/\eta_0}{C}$$

wherein η is a value as measured using an Ubbellohde viscometer at an oligomer concentration of 0.5 g/dl in N-methyl-2-pyrrolidone at a temperature of 30 ± 0.01°C, $\eta_0$ is the value for N-methyl-2-pyrrolidone as measured using the same viscometer at the same temperature, and C represents the oligomer concentration of 0.5 g/dl; and represented by the following general formula (I):

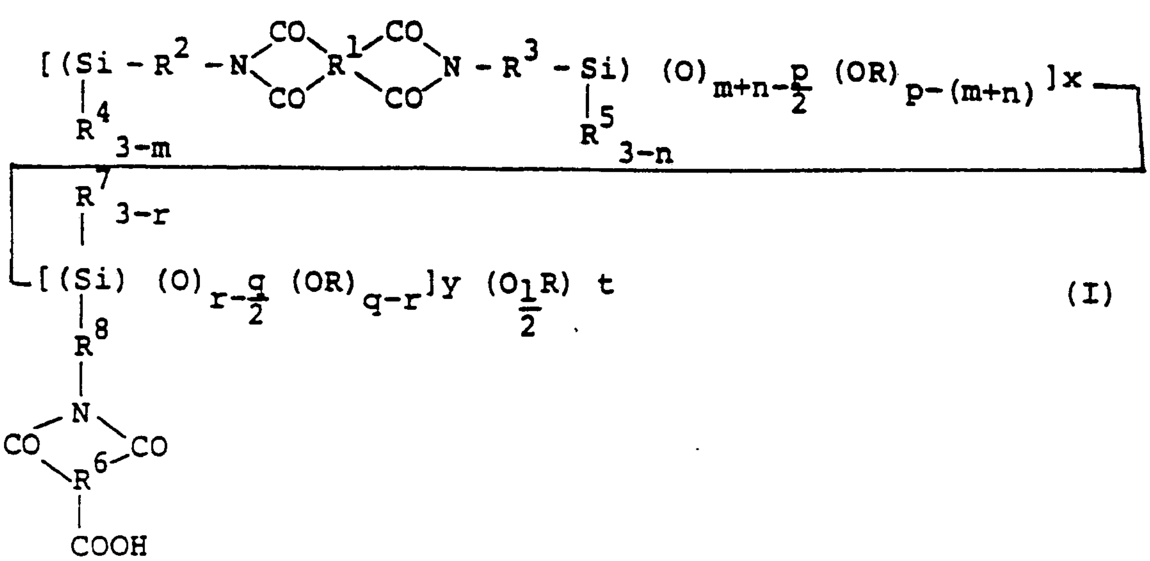

(I)

wherein

$R^1$ and $R^6$ are tetravalent and trivalent carbocyclic aromatic groups, respectively, the carbonyl groups being bonded to the carbocyclic aromatic groups at mutually different ring-forming carbon atoms thereof, every pair of imide ring-forming carbonyl groups being bonded to a carbocyclic ring of said carbocyclic aromatic groups in the mutual ortho positions thereof;

$R^2$, $R^3$ and $R^8$, which are the same or different groups, are selected from

$-(CH_2)_s-$ , 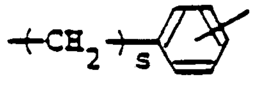 , 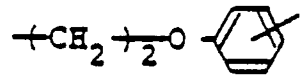 , or 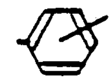

(wherein *s* is an integer of 1 to 4);

$R^4$, $R^5$ and $R^7$, which are the same or different groups, are an alkyl group having 1 to 6 carbon atoms, a phenyl group, or an alkyl-substituted phenyl group having 7 to 12 carbon atoms;

R is hydrogen, alkyl or acetyl;

*m* and *n* each is an integer of 1, 2 or 3, and satisfy the equation: $m + n \geqq 3$;

*r* is 2 or 3; and

*p* and *q* are integers and satisfy the relations: $m + n \leqq p < 2(m + n) - 1$ and $r \leqq q < 2r - 1$;

*t* is 0 or 2; and

*x* and *y* are values satisfying the following equation:

$$0 \leqq \frac{100\,y}{x + y} \leqq 10.$$

2. A soluble imide oligomer as claimed in claim 1, wherein *x* and *y* are values satisfying the following equation:

$$0.1 \leqq \frac{100\,y}{x + y} \leqq 10.$$

3. A soluble imide oligomer as claimed in claim 1 or 2, wherein $R^1$ is

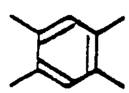 , 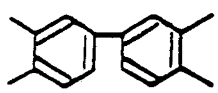 or 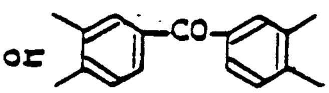

4. A soluble imide oligomer as claimed in claim 1, 2 or 3, wherein $R^6$ is

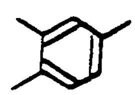

5. A soluble imide oligomer as claimed in any preceding claim, wherein $R^2$, $R^3$ and $R^8$ each is

$-(CH_2)_3-$, or 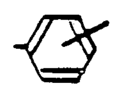

6. A soluble imide oligomer as claimed in any preceding claim, wherein $R^4$, $R^5$ and $R^7$ each is

$-CH_3$, $-C_2H_5$, or 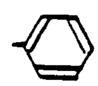

7. A process for preparing a soluble imide oligomer as claimed in any one of the preceding claims which process comprises reacting an amic acid compound represented by the general formula (II):

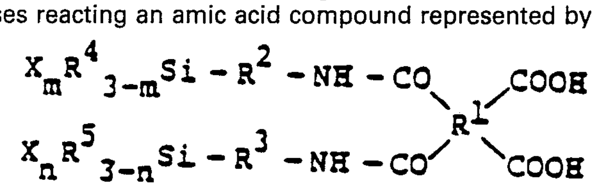 (II)

wherein $R^1$, $R^2$, $R^3$, $R^4$, $R^5$, *m* and *n* are as defined in claim 1 and X is a halogen atom or an alkoxy, acetoxy or hydroxy group, or a mixture thereof with 10% by mole or less of an amic acid compound represented by the general formula (III) based on the total amount of compound (II) and (III):

$$X_rR^7_{3-r}Si—R^8—NH—CO—R^6\!\!-\!\!(COOH)_2 \qquad (III),$$

wherein $R^6$, $R^7$, $R^8$, and *r* are as defined in claim 1, is heated in the presence of 70% by weight or more (based on the solution) of a solvent, at a temperature of 60 to 200°C for 0.2 to 6 hours.

8. A process according to claim 7, wherein the heating is effected in the presence of water.

9. A process according to claim 8, wherein the amount of water is $\{(m + n - 2)x + (r - 1)y\}$ mole or less per mole of the amic acid compound.

10. A process according to claim 7, 8 or 9, wherein the solvent includes 30 to 100% by weight of at least one of N-methyl-2-pyrrolidone, dimethylacetamide, dimethylformamide, toluene, xylene, methoxy ethanol, or dimethyl sulphoxide.

11. A coating solution for electronic materials, the coating solution comprising a solvent and a soluble imide oligomer, the soluble imide oligomer being present in an amount of 30% by weight or less (based on the total of the solvent and the soluble imide oligomer) and being in accordance with any of claims 1 to 6.

12. A coating solution as claimed in claim 11, wherein the solvent includes 30 to 100% by weight of at least one of N-methyl-2-pyrrolidone, dimethylacetamide, dimethylformamide, toluene, xylene, methoxy ethanol, 2-ethoxy-ethanol, or dimethyl sulphoxide.

**Patentansprüche**

1. Lösliches Imid-Oligomer mit einer logarithmischen Viskositätszahl ($\eta_{inh}$) von 0,05 bis 0,5 dl/g, bestimmt nach folgender Gleichung:

$$\eta\ inh = \frac{\ln \eta/\eta_0}{C}$$

worin η ein unter Verwendung eines Ubberlohde-Viskosimeters bei einer Oligomer-Konzentration von 0,5 g/dl in N-Methyl-2-pyrrolidon bei einer Temperatur von 30 ± 0,01°C gemessener Wert ist, $\eta_0$ der für N-Methyl-2-pyrrolidon unter Verwendung desselben Viskosimeters bei derselben Temperatur gemessene Wert ist und C die Oligomer-Konzentration von 0,5 g/dl bedeutet, und wobei das Oligomer der folgenden allgemeinen Formel (I) entspricht:

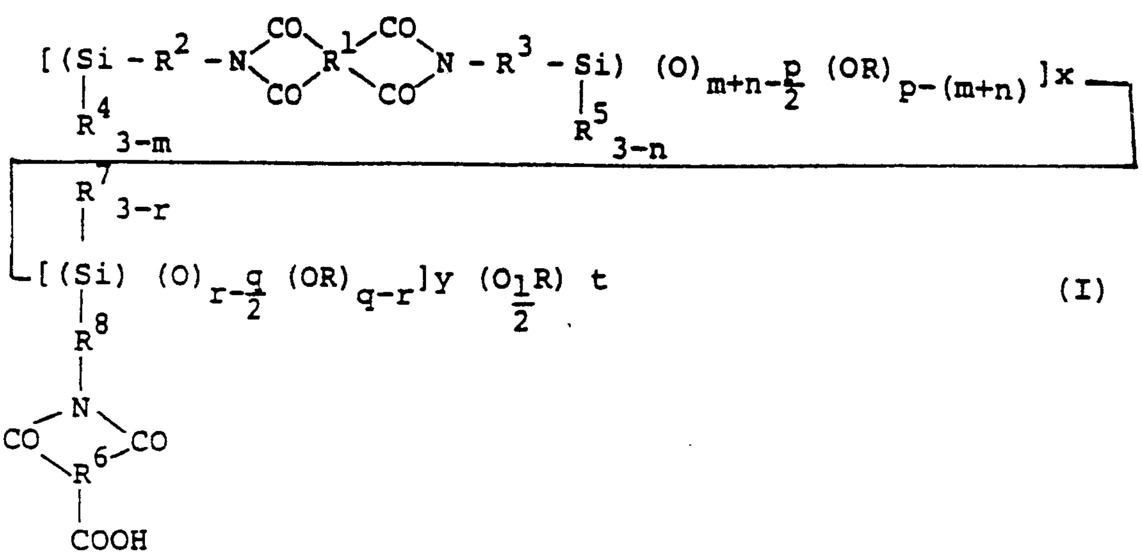

(I)

worin

$R^1$ und $R^6$ vierbindige bzw. dreibindige carbocyclische aromatische Gruppen sind und die Carbonylgruppen an jeweils verschiedene ringbildende Kohlenstoffatome der carbocyclischen aromatischen Gruppen gebunden sind, wobei jedes Paar von imidringbildenden Carbonylgruppen an

einen carbocyclischen Ring der carbocyclischen aromatischen Gruppen jeweils in desen ortho-Stellung gebunden ist;

$R^2$, $R^3$ und $R^8$, die gleich oder verschieden sind, ausgewählt sind aus den Gruppen

$-(CH_2)_s-$, 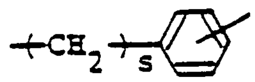 , 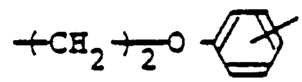 , or 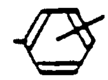

(worin *s* eine ganze Zahl von 1 bis 4 ist);

$R^4$, $R^5$ und $R^7$, die gleich oder verschieden sind, jeweils eine Alkylgruppe mit 1 bis 6 Kohlenstoffatomen, eine Phenylgruppe oder eine alkylsubstituierte Phenylgruppe mit 7 bis 12 Kohlenstoffatomen bedeuten;

R Wasserstoff, Alkyl oder Acetyl ist;

*m* und *n* jeweils eine ganze Zahl von 1 bis 3 bedeuten und der Gleichung $m + n \geqq 3$ gehorchen;

*r* die Zahl 2 oder 3 ist; und

*p* und *q* ganze Zahlen sind und den Gleichungen $m + n \leqq p < 2(m + n) - 1$ und $r \leqq q < 2r - 1$ gehorchen);

*t* die Zahl 0 oder 2 ist; und

*x* und *y* Werte sind, die der folgenden Gleichung gehorchen:

$$0 \leqq \frac{100\,y}{x + y} \leqq 10$$

2. Lösliches Imid-Oligomer nach Anspruch 1, worin *x* und *y* Werte sind, die der folgenden Gleichung gehorchen:

$$0.1 \leqq \frac{100\,y}{x + y} \leqq 10$$

3. Lösliches Imid-Oligomer nach Anspruch 1 oder 2, worin $R^1$

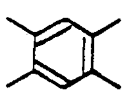 , 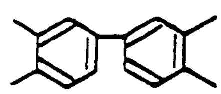 or 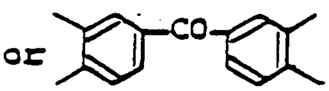

bedeutet.

4. Lösliches Imid-Oligomer nach den Ansprüchen 1, 2 oder 3, worin $R^6$

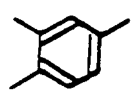

bedeutet.

5. Lösliches Imid-Oligomer nach einem der vorhergehenden Ansprüches, worin $R^2$, $R^3$ und $R^8$ jeweils

$-(CH_2)_3-$, or 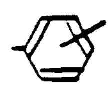

bedeuten.

6. Lösliches Imid-Oligomer nach einem der vorhergehenden Ansprüche, worin $R^4$, $R^5$ und $R^7$ jeweils

$-CH_3$, $-C_2H_5$, or 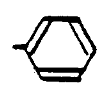

bedeuten.

7. Verfahren zur Herstellung eines löslichen Imid-Oligomers gemäß einem der vorhergehenden Ansprüche, bei dem eine Säureamid-Verbindung der allgemeinen Formel (II):

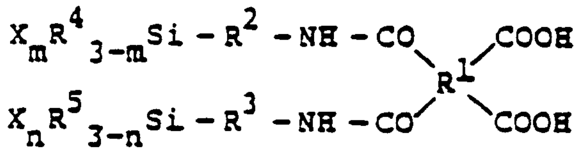 (II)

worin $R^1$, $R^2$, $R^3$, $R^4$, $R^5$, *m* und *n* die in Anspruch 1 genannten Bedeutungen besitzen und X ein Halogenatom oder eine Alkoxy-, Acetoxy- oder Hydroxy-Gruppe ist, oder ein Gemisch dieser Säureamid-Verbindung mit 10 Mol-% oder weniger einer Säureamid-Verbindung der allgemeinen Formel (III), bezogen auf die Gesamtmenge der Verbindungen (II) und (III):

$$X_rR^7_{3-r}Si{-}R^8{-}NH{-}CO{-}R^6{-}(COOH)_2 \qquad (III)$$

worin $R^6$, $R^7$, $R^8$ und *r* die in Anspruch 1 genannten Bedeutungen besitzen, in Gegenwart von 70 Gew.% oder mehr (bezogen auf die Lösung) eines Lösungsmittels erhitzt und bei einer Temperatur von 60 bis 200°C 0,2 bis 6 Stunden lang umgesetzt wird.

8. Verfahren nach Anspruch 7, bei dem das Erhitzen in Gegenwart von Wasser durchgeführt wird.

9. Verfahren nach Anspruch 8, worin die Menge an Wasser $\{(m + n - 2)x + (r - 1)y\}$ Mol oder weniger pro Mol der Säureamid-Verbindung beträgt.

10. Verfahren nach einem der Ansprüche 7, 8 oder 9, bei dem das Lösungsmittel 30 bis 100 Gew.% an mindestens einem der folgenden einschließt: N-Methyl-2-pyrrolidon, Dimethylacetamid, Dimethylformamid, Toluol, Xylol, Methoxyethanol oder Dimethylsulfoxid.

11. Beschichtungslösung für elektronische Materialien, wobei die Beschichtungslösung ein Lösungsmittel und ein lösliches Imid-Oligomer umfaßt und wobei das lösliche Imid-Oligomer in einer Menge von 30 Gew.% oder weniger vorliegt (bezogen auf die Gesamtmenge an Lösungsmittel und löslichem Imid-Oligomer) und einem der Ansprüche 1 bis 6 entspricht.

12. Beschichtungslösung nach Anspruch 11, worin das Lösungsmittel 30 bis 100 Gew.% mindestens eines der folgenden einschließt: N-Methyl-2-pyrrolidon, Dimethylacetamid, Dimethylformamid, Toluol, Xylol, Methoxyethanol, 2-Ethoxyethanol oder Dimethylsulfoxid.

**Revendications**

1. Oligomère d'imide soluble présentant une viscosité inhérente (η inh) de 0,05 à 0,5 dl/g qui est définie par l'équation suivante:

$$\eta\ inh = \frac{\ln \eta/\eta_0}{C}$$

dans laquelle n et une valeur que l'on mesure au moyen d'un viscosimètre Ubbelohde à une concentration oligomère de 0,5 g/dl dans de la N-méthyl-2-pyrrolidone à une température de 30 ± 0,01°C, $\eta_0$ est la valeur de la N-méthy-2-pyrrolidone que l'on mesure au moyen du même viscosimètre, à la même température, et C représente la concentration oligomère de 0,5 g/dl, et qui répond à la formule générale suivante:

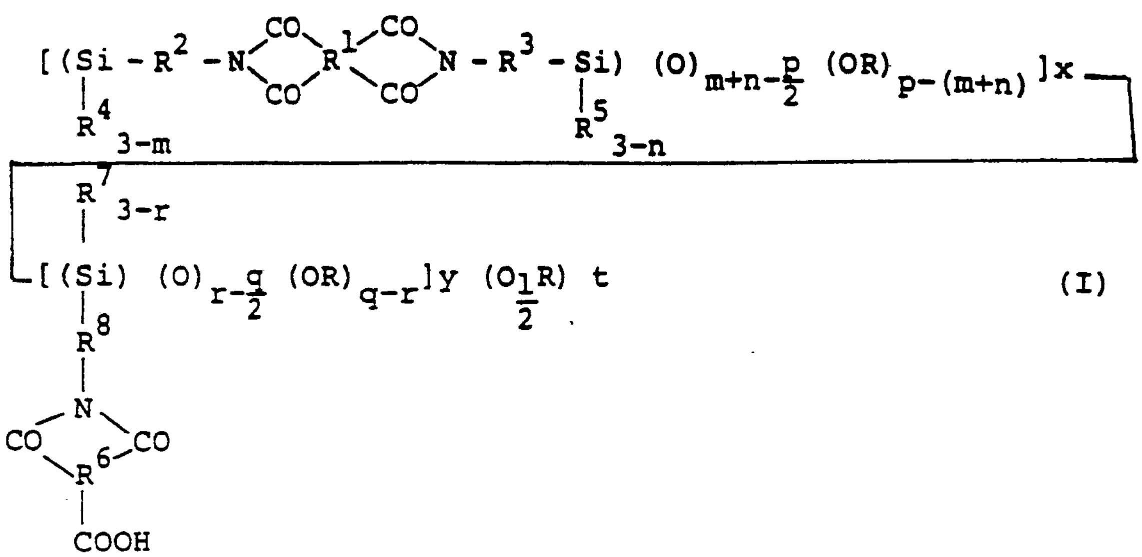

(I)

dans laquelle

$R^1$ et $R^6$ représentent des groupes aromatiques isocycliques respectivement tétravalent et trivalent, les groupes carbonyle étant reliés aux groupes aromatiques isocycliques à des atomes de carbone cyclisant de ces derniers, différents les uns des autres, chaque paire de groupes carbonyle cyclisant d'imide étant reliée

à un composé isocyclique desdits groupes aromatiques isocycliques aux positions ortho réciproques de ceux-ci;

$R^2$, $R^3$ et $R^8$, qui sont des groupes identiques ou différents, sont choisis parmi les groupes suivants:

$-(CH_2)_s-$ , 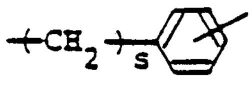 , 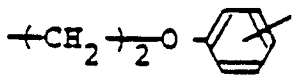 , or 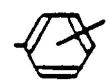

(où $s$ est un nombre entier valant de 1 à 4);

$R^4$, $R^5$ et $R^7$, qui sont des groupes identiques ou différents, représentent und groupe alkyle ayant de 1 à 6 atomes de carbone, un groupe phényle ou un groupe alkyle-substitué phényle ayant de 7 à 12 atomes de carbone;

R représente l'hydrogène, un alkyle ou un acétyle;

$m$ et $n$ représentent chacun un nombre entier valant 1, 2 ou 3 et répondent à l'équation $m + n \geqslant 3$;

$r$ vaut 2 ou 3; et

$p$ et $q$ représentent chacun un nombre entier et répondent aux relations: $m + n \leqslant p < (m + n) -1$ et $r \leqslant q < 2r - 1$;

$t$ vaut ou 2; et

$x$ et $y$ sont des valeurs répondant à l'équation suivante:

$$0 \leqq \frac{100\,y}{x + y} \leqq 10.$$

2. Oligomère d'imide soluble selon la revendication 1, caractérisé en ce que $x$ et $y$ sont des valeurs qui répondent à l'équation suivante:

$$0{,}1 \leqq \frac{100\,y}{x + y} \leqq 10.$$

3. Oligomère d'imide soluble selon la revendication 1 ou 2, caractérisé en ce que $R^1$ représente:

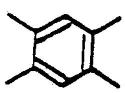 , 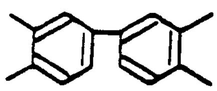 or 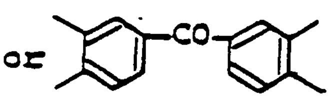

4. Oligomère d'imide soluble selon la revendication 1, 2 ou 3, caractérisé en ce que $R^6$ représente:

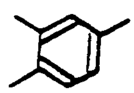

5. Oligomère d'imide soluble selon l'une quelconque des revendications précédentes, caractérisé en ce que $R^2$, $R^3$ et $R^8$ représentent chacun:

$-(CH_2)_3-$, or 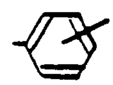

6. Oligomère d'imide soluble selon l'une quelconque des revendications précédentes, caractérisé en ce que $R^4$, $R^5$ et $R^7$ représentent chacun:

$-CH_3$, $-C_2H_5$, or 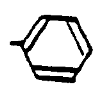

7. Procédé de préparation d'un oligomère d'imide soluble selon l'une quelconque des revendications précédentes, caractérisé en ce qu'on fait réagir un composé d'amide d'acide, qui a répond à la formule générale (II):

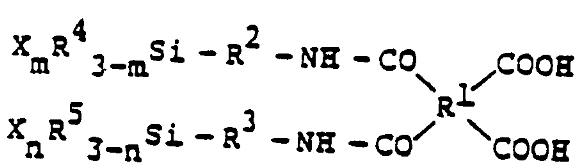

(II).

dans laquelle $R^1$, $R^2$, $R^3$, $R^4$, $R^5$, *m* et *n* sont définis comme dans la revendication 1 et X représente un atome d'halogène ou un groupe alcoxy, acétoxy ou hydroxy, ou un mélange de ceux-ci, avec 10% en mole, ou moins, d'un composé d'amide d'acide répondant à la formule générale (III), par rapport à la quantité totale des composés (II) et (III):

$$X_rR^7_{3-r}Si—R^8—NH—CO—R^6\text{+}COOH)_2 \qquad (III),$$

dans laquelle $R^6$, $R^7$, $R^8$ et *r* sont définis comme dans la revendication 1, on chauffe le mélange réactionnel à une température comprise entre 60 et 200°C, pendant 0,2 à 6 heures, en présence d'un solvant contenu à raison de 70% en poids ou plus (par rapport à la solution).

8. Procédé selon la revendication 7, caractérisé en ce que l'on effectue le chauffage en présence d'eau.

9. Procédé selon la revendication 8, caractérisé en ce que la quantité d'eau est de $[(m + n - 2)x + (r - 1)y]$ mole, ou moins, par mole de composé d'amide d'acide.

10. Procédé selon l'une des revendications 7 à 9, caractérisé en ce que le solvant contient de 30 à 100% en poids d'au moins un des composés suivants: N-méthyl-2-pyrrolidone, diméthylacétamide, diméthylformamide, toluène, xylène, méthoxy éthanol ou diméthyle sulfoxyde.

11. Solution de revêtement pour matériaux électroniques, contenant un solvant et un oligomère d'imide souble selon l'une quelconque des revendications 1 à 6, ledit oligomère d'imide soluble représentant 30% en poids, ou moins, de la quantité totale du solvant et de l'oligomère d'imide soluble.

12. Solution de revêtement selon la revendication 11, caractérisée en ce que le solvant contient de 30 à 100% en poids d'au moins un des composés suivants: N-méthyl-2-pyrrolidone, diméthylacétamide, diméthylformamide, toluène, xylène, méthoxy éthanol, 2-éthoxy-éthanol ou diméthyle sulfoxyde.

FIG. I

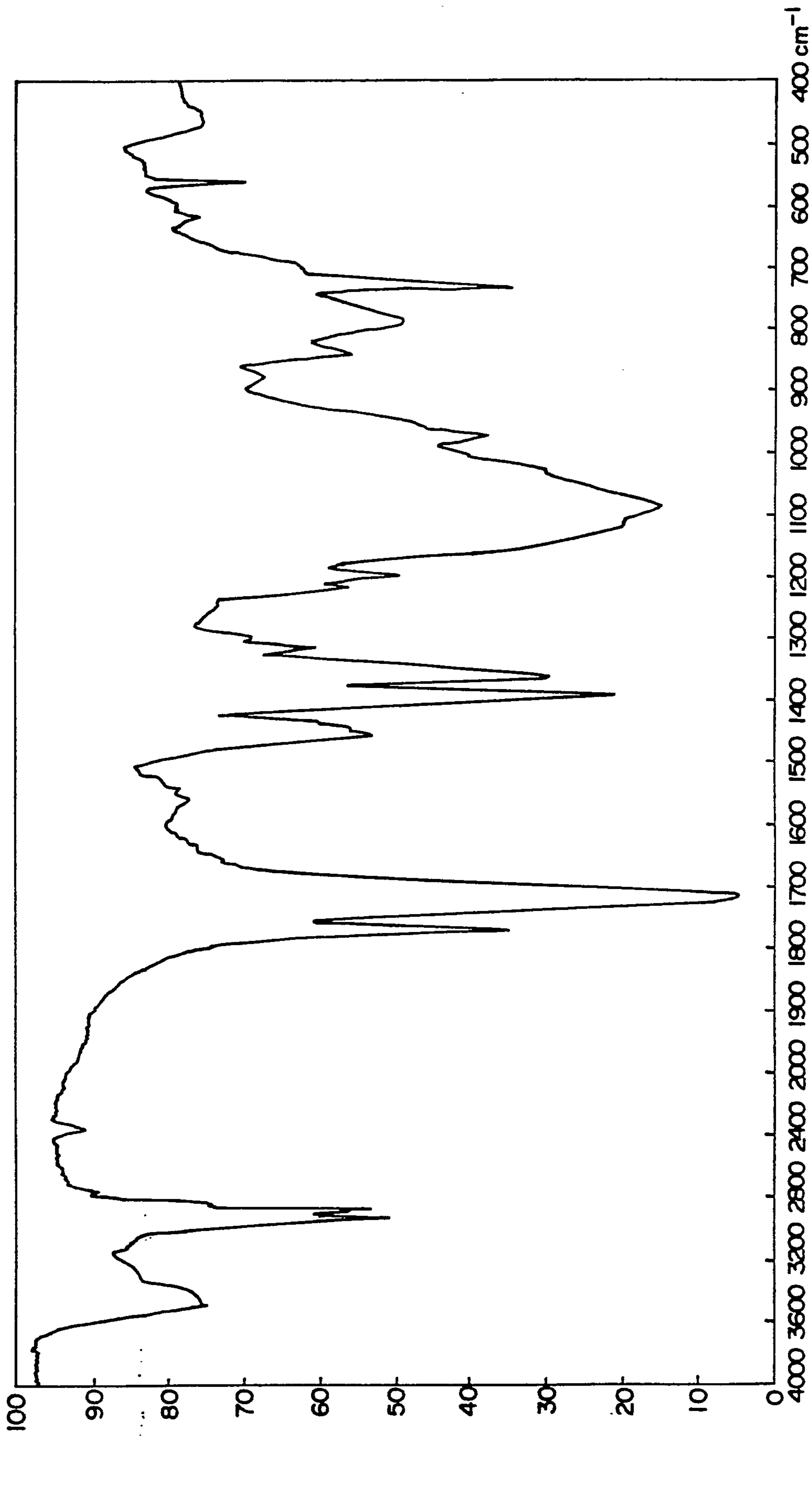
100
90
80
70
60
50
40
30
20
10
0
4000
3600
3200
2900
2400
2000
1900
1800
1700
1600
1500
1400
1300
1200
1100
1000
900
800
700
600
500
400 cm⁻¹